# FASCICULE DE BREVET EUROPEEN

(11) **EP 3 615 038 B1**
(45) Date de publication et mention de la délivrance du brevet: **15.09.2021**
(21) Numéro de dépôt: 18723360.6
(22) Date de dépôt: 26.04.2018
(51) Int. Cl.: A61K 31/702, A61P 1/00

(54) **COMPOSITION NUTRACEUTIQUE ET/OU PHARMACEUTIQUE POUR STIMULER LA PRODUCTION DE BETA-DEFENSINES DE TYPE 2**
NUTRAZEUTISCHE UND/ODER PHARMAZEUTISCHE ZUSAMMENSETZUNG ZUR STIMULIERUNG DER PRODUKTION VON BETA-DEFENSINEN 2
NUTRACEUTICAL AND/OR PHARMACEUTICAL COMPOSITION FOR STIMULATING THE PRODUCTION OF BETA-DEFENSINS-2

(30) Priorité: 26.04.2017 FR 1770423
(43) Date de publication de la demande: 04.03.2020
(73) Titulaire: Usines Chimiques D'Ivry La Bataille, 28260 Anet (FR)
(72) Inventeur: SAGUET, Thibaut, 28260 Anet (FR); LASALLE, Laurent, 28260 Anet (FR); PHILBE, Jean-Luc, 28260 Anet (FR); YVERGNAUX, Florent, 28260 Anet (FR)
(74) Mandataire: Célanie, Christian
(86) Numéro de dépôt international: PCT/FR2018/000101
(87) Numéro de publication internationale: WO 2018/197762

(56) Documents cités:
- WO-A1-2014/092564
- FR-A1- 2 996 138
- Anonymous: "Full Public Report - Bioecolia (File N°: NA/676)", National Industrial Chemicals Notification and Assessment Scheme , juin 1999 (1999-06), XP002777331, Extrait de l'Internet: URL:https://www.nicnas.gov.au/__data/asset s/word_doc/0009/20403/NA676FR.docx [extrait le 2017-11-17]
- "Alpha-Glucan oligosaccharide; Inulin ED - Gottschalck Tara E; Bailey John E", 1 janvier 2008 (2008-01-01), INTERNATIONAL COSMETIC INGREDIENT DICTIONARY AND HANDBOOK ; VOL. 1, THE COSMETIC, TOILETRY, AND FRAGRANCE ASSOCIATION, WASHINGTON, D.C, PAGE(S) 1039,1283, XP002723826, page 1, colonne de gauche
- WEHKAMP JAN ET AL: "NF-kappaB- and AP-1-mediated induction of human beta defensin-2 in intestinal epithelial cells by Escherichia coli Nissle 1917: A novel effect of a probiotic bacterium", INFECTION AND IMMUNITY, vol. 72, no. 10, octobre 2004 (2004-10), pages 5750-5758, XP002777332, ISSN: 0019-9567, DOI: 10.1128/IAI.72.10.5750-5758.2004

## Description

Le secteur technique de la présente invention est celui des compositions nutraceutiques ou pharmaceutiques destinées à réduire les inflammations de l'appareil digestif et à limiter la prolifération de certains microorganismes dans l'intestin.

L'intestin est un environnement complexe et fragile où de nombreux processus physiologiques se déroulent. C'est un milieu dans lequel vivent et prolifèrent de nombreuses populations de microorganismes. Les perturbations de ces équilibres provoquent des maladies et des désordres divers de l'organisme.

De nombreux traitements sont utilisés pour éviter, traiter ou amoindrir ces maladies avec, souvent, des effets nocifs sur les populations commensales vivant dans l'intestin, ce qui provoque d'autres symptômes négatifs qui doivent à leur tour être traités.

On sait depuis un certain nombre d'années que les probiotiques et prébiotiques favorisent un bon confort intestinal et permettent aussi de lutter contre certains problèmes liés à l'intestin (Rapport AFSSA, Boclé et Thomann, février 2005). Les prébiotiques sont représentés notamment par les fructo-oligosaccharides et les galacto-oligosaccharides.

Par exemple, on a montré l'intérêt des fructo-oligosaccharides pour agir sur la flore intestinale, réduire l'obésité et le diabète de type 2 (ISME Journal, 2014, 8, 2116) .

En ciblant et en augmentant la quantité intestinale de certaines familles de bactéries comme *Akkermancia muciniphila,* il est aussi possible de lutter encore plus efficacement contre l'obésité (Brevet WO2014075745). Des inflammations intestinales peuvent être réduites par absorption d'un mélange constitué d'un probiotique du type *Bifidobactérium longum* associé à un prébiotique de type fructo-oligosaccharide (British Journal of Nutrition, 2005, 93S1, S67).

On a également montré dans la sphère de l'immunité que l'association d'un prébiotique avec un probiotique présente un potentiel d'action sur l'immunité. Par exemple, dans une étude conduite sur des brèmes de mer, une nourriture supplémentée en prébiotique du type fructo-oligosaccharides et en probiotique du type *Bacillus subtilis* entraine une nette diminution de la mortalité de ces poissons par rapport au groupe placebo, ce qui démontre un lien avec une meilleure immunité (Fish and Shellfish Immunology, 2012, 32, 1032). De plus, une étude menée sur des enfants recevant une supplémentation alimentaire avec un prébiotique du type fructo-oligosaccharides et galacto-oligosaccharide a montré que ces enfants présentaient de meilleures défenses immunitaires. Cependant, lors de cette étude, on avait noté une diminution des alpha-défensines salivaires contrairement aux groupes témoins (Pediatria Polska, 2013, 88, 398).

Les défensines sont une famille de molécules peptidiques qui possèdent des propriétés antimicrobiennes chez l'Homme. Il existe les alpha-défensines et les béta-défensines dont le rôle est par exemple de renforcer l'immunité ou bien encore de limiter la prolifération de certaines bactéries.

Parmi les béta-défensines, il existe la béta-défensine de type 2 humaine qui est particulièrement importante d'un point de vue biologique et qui est codée par le gène DEFB4 (defensin, beta 4). Elle présente un potentiel bactéricide contre des bactéries Gram négatif et contre les champignons du type *Candida.* Elle fait-aussi partie du système de défense de l'immunité innée. Elle contribue à maintenir un bon équilibre au sein du microbiote humain.

Les prébiotiques du type galacto-oligosaccharides ou fructo-oligosaccharides ont montré récemment un potentiel de favorisation de la production de ces béta-défensines. Ainsi, il a été décrit lors d'une étude sur des porcelets que l'utilisation dans la nourriture de galacto-oligosaccharides favorisait la production de beta-défensine de type-2 au niveau du colon de ces porcelets (British Journal of Nutrition, 2016, 115, 605).

Une autre étude montre que des porcelets dont l'alimentation est supplémentée en probiotique de type *Lactobacillus reuteri* et *Pediococcus pentosaceus* ainsi qu'en topinambour qui est riche en fructo-oligosaccharide présentaient une augmentation intestinale de la production de béta-défensines des types 2 et 3 (Polish Journal of Veterinary Sciences, 2014, 17, 61).

Une étude en double aveugle avec une supplémentation en prébiotique de type *Bifidobacterium longum* et des fructo-oligosaccharides a montré une augmentation de la production de béta-défensine de type 2, 3 et 4 (GUT, 2005, 54, 242).

Dans le brevet FR-2996138, on a décrit l'utilisation d'un gluco-oligosaccharide dans la production cutanée de peptides antimicrobiens pour des applications dermo-cosmétiques.

La littérature examinée ci-dessus montre tout l'intérêt des pré- et probiotiques pour améliorer le confort intestinal. Toutefois, l'utilisation des oligosaccharides n'a pas été complètement explorée et on a constaté que le remplacement d'un oligosaccharide par un autre ne procurait pas obligatoirement le même effet.

Ainsi, les auteurs d'une étude conduite chez les rats ont montré différentes actions métaboliques en fonction des prébiotiques ingérés. Il en avait été conclu que les oligosaccharides étudiés (fructo-oligosaccharides, galacto-oligosaccharides et gluco-oligosaccharides) avaient différents effets métaboliques, à relier à leur structure chimique (British Journal of Nutrition, 1997, 78, 313). Plus récemment une étude comparative à partir de souches intestinales en contact avec deux sortes de prébiotiques (gluco-oligosaccharides et fructo-oloigosaccharides) a montré des différences métaboliques significatives (British Journal of Nutrition, 2013, 109, 1980). En 2000, ce type d'étude avait été conduite sur des chiens et il avait été aussi montré qu'une modification de la structure chimique du prébiotique administré entrainait des résultats biologiques différents (Am. Soc. For Nutr. Sci. 2000, 1267).

Ainsi le but de l'invention est de fournir un traitement intéressant parfaitement bien supporté par l'organisme destiné à stimuler la production de béta-défensine de type 2 par l'organisme et, par conséquent, à provoquer une réduction ou une disparition des problèmes de colopathie fonctionnelle.

L'invention a donc pour objet une composition nutraceutique et/ou pharmaceutique destinée à stimuler la production de béta-défensine de type 2, caractérisée en ce qu'elle comprend à titre de principe actif unique un gluco-oligosaccharide répondant à la structure de base oligomérique suivante : 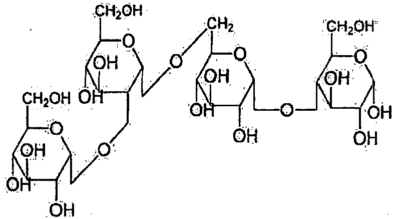 pour le traitement des inflammations intestinales.

Selon une caractéristique de l'invention, la composition comprend entre 0,5 et 10 g de gluco-oligosaccharide.

Avantageusement, la composition comprend 1 g de gluco-oligosaccharide.

Avantageusement encore, la composition comprend 2,5 g de gluco-oligosaccharide.

Avantageusement encore, la composition comprend 4 g de gluco-oligosaccharide.

Avantageusement encore, la composition comprend 6 g de gluco-oligosaccharide.

Avantageusement encore, la composition comprend 9,5 g de gluco-oligosaccharide.

L'invention a en outre pour objet l'utilisation de la composition pour réduire ou traiter une colopathie fonctionnelle.

L'invention a également pour objet l'utilisation de la composition à titre de complément alimentaire.

La composition selon l'invention se présente sous la forme de comprimés ou de gélules administrés par dose une fois par jour.

Un avantage de la composition pharmaceutique selon l'invention est qu'elle ne comporte pas de composant agressif pour les microorganismes intestinaux.

Un autre avantage réside dans le fait qu'elle ne détruit pas de populations microbiennes bénéfiques pour l'organisme simultanément à l'élimination des populations nocives.

Un autre avantage de la présente invention réside dans l'absence de nocivité de la composition même en cas de surdosage.

Un autre avantage encore de la présente invention réside dans l'activité surprenante du gluco-oligosaccharide utilisé seul comme principe actif.

Un autre avantage encore de la présente invention réside dans une action directe au niveau de la muqueuse intestinale.

D'autres caractéristiques, avantages et détails de l'invention seront mieux compris à la lecture du complément de description qui va suivre.

Les infections intestinales sont dues en partie à des déséquilibres dans la flore intestinale qui favorisent des microorganismes pathogènes, au détriment des populations commensales. Ces déséquilibres ont des causes multiples parmi lesquelles on peut citer le stress, la fatigue, l'alimentation et l'état psychologique.

Les prébiotiques sont des oligosaccharides qui favorisent le développement des microorganismes bénéfiques à l'organisme. En effet, leur structure les rend indigestibles par l'appareil digestif. Ils arrivent donc intacts dans le côlon, où ils sont une source alimentaire pour les microorganismes commensaux de la flore intestinale. Leur supplémentation dans l'alimentation est connue pour améliorer le confort digestif et réduire les épisodes infectieux.

Le gluco-oligosaccharide de l'invention est un prébiotique de ce type, déjà utilisé pour ses propriétés cosmétiques sur la peau et pour améliorer le confort intestinal. Le confort intestinal s'entend comme l'état « normal » de fonctionnement de l'appareil digestif, qui est perturbé par la colopathie fonctionnelle, communément appelée syndrome du côlon irritable, qui est une anomalie de fonctionnement du tube digestif.

La colopathie fonctionnelle est un trouble de fonctionnement du côlon. Le côlon est une partie de l'intestin située entre l'intestin grêle et le rectum. Il termine la digestion des aliments, débutée dans l'intestin grêle, et participe à la formation et l'évacuation des selles.

Due à une hypersensibilité du côlon, le syndrome du côlon irritable est à l'origine de douleurs abdominales, de constipation, de diarrhée et de ballonnements. Bien que bénin, ce syndrome peut se manifester de façon chronique et entraîner une dégradation de la qualité de vie.

En approfondissant leurs études, les inventeurs ont découvert l'effet surprenant et inattendu de ce gluco-oligosaccharide, utilisé seul, résidant dans son action de stimulation immunitaire en favorisant la production de béta-défensine de type 2.

En effet, lors de l'ingestion du gluco-oligosaccharide, en dehors de son utilisation comme aliment par les microorganismes présents dans l'intestin, ce dernier entre en contact avec les cellules épithéliales de l'intestin. Ce contact provoque, dans ces cellules de l'organisme, une augmentation de la production de béta-défensine de type 2.

La béta-défensine 2 est un peptide de la famille des défensines. Elle fait partie du système immunitaire où elle joue deux rôles. D'une part, elle diminue l'inflammation, d'autre part, elle est capable de détruire certains germes, en l'occurrence les bactéries Gram- et les champignons du type Candida. La béta-défensine de type 2 fait partie des PAM, les peptides antimicrobiens, qui sont des peptides microbicides.

Le gluco-oligosaccharide selon l'invention a donc un effet thérapeutique d'une part sur les inflammations telles que le syndrome du côlon irritable, et d'autre part sur les infections, en stimulant la production de béta-défensine de type 2.

On a donc réalisé une étude sur un panel de 50 personnes présentant des symptômes du côlon irritable.

Cette étude a été réalisée de manière monocentrique avec deux groupes en parallèle (le groupe avec le gluco-oligosaccharide et le groupe placebo) randomisée, en double aveugle et avec un contrôle placebo. Les 50 sujets étaient des adultes présentant un syndrome de côlon irritable associé à de la constipation. Ils ont été répartis de manière aléatoire dans deux groupes de 25 volontaires : un groupe traité avec le gluco-oligosaccharide et un groupe traité avec un placebo (du glucose). La sécurité et l'efficacité du produit ont été évaluées sur une étude avec une durée de traitement fixée à 28 jours.

Au cours d'une visite de pré inclusion, on a investigué et sélectionné des sujets éligibles à l'étude à partir d'un questionnaire sur les symptômes de côlon irritable et sur les habitudes alimentaires.

Les prélèvements de selles ont été réalisés à l'aide d'un kit au début de l'étude, au jour 0 et à la fin de l'étude, au 28ème jour, pour la réalisation de différents dosages dont celui en particulier de la béta-défensine de type 2. Entre le jour 0 et le 28ème jour, un groupe de volontaires a pris une dose quotidienne de 2 g de gluco-oligosaccharide et l'autre groupe une dose quotidienne de 2g de glucose en guise de placébo.

Le traitement a été très bien toléré par tous les sujets de l'étude aucun effet négatif n'a été rapporté.

Les résultats obtenus sont décrits ci-dessous :
La production de béta-défensine de type 2 a été caractérisée par les inventeurs par la méthode ELISA (Enzyme-linked immunosorbent assay).
Evaluation de la quantité de bêta-défensine 2 (en ng / g de selle).

| **Produits** | **Jour 0** | **Jour 28** | **Significativité** |
|---|---|---|---|
| Gluco-oligosaccharide | 51,28 | 62, 33 | p < 0,05 |
| Placebo | 49, 77 | 54, 52 | non |
| (glucose) | | | significatif |

Au cours de l'étude, on a mesuré une augmentation significative de la production de béta-défensine de type 2 intestinale dans le groupe traité avec le gluco-oligosaccharide entre le 1^{er} jour de traitement et le 28ème jour, alors qu'il n'y pas eu d'augmentation significative pour le groupe traité avec le placebo. Cette augmentation pour le groupe traité avec le gluco-oligosaccharide traduit une augmentation des défenses de l'immunité innée de la muqueuse intestinale avec des applications potentielles dans un certain nombre de problématiques intestinales.

Aucun effet secondaire n'a été constaté. L'utilisation du gluco-oligosaccharide ouvre donc une voie nouvelle pour le traitement de patients souffrant de la colopathie fonctionnelle sans aucune atteinte de la muqueuse intestinale.

## Revendications

1. Composition nutraceutique et/ou pharmaceutique destinée à stimuler la production de béta-défensines de type 2, **caractérisée en ce qu'**elle comprend à titre de principe actif unique un gluco-oligosaccharide répondant à la structure de base oligomérique suivante : 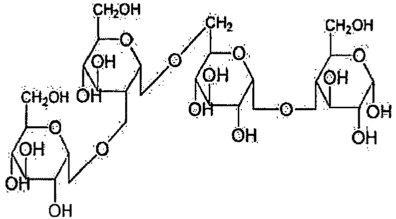 pour son utilisation dans le traitement des inflammations intestinales.

2. Composition nutraceutique et/ou pharmaceutique pour son utilisation selon la revendication 1, **caractérisée en ce qu'**elle comprend entre 0,5 et 10 g de gluco-oligosaccharide.

3. Composition nutraceutique et/ou pharmaceutique pour son utilisation selon la revendication 2, **caractérisée en ce qu'**elle comprend 1 g de gluco-oligosaccharide.

4. Composition nutraceutique et/ou pharmaceutique pour son utilisation selon la revendication 2, **caractérisée en ce qu'**elle comprend 2,5 g de gluco-oligosaccharide.

5. Composition nutraceutique et/ou pharmaceutique pour son utilisation selon la revendication 2, **caractérisée en ce qu'**elle comprend 4 g de gluco-oligosaccharide.

6. Composition nutraceutique et/ou pharmaceutique pour son utilisation selon la revendication 2, **caractérisée en ce qu'**elle comprend 6 g de gluco-oligosaccharide.

7. Composition nutraceutique et/ou pharmaceutique pour son utilisation selon la revendication 2, **caractérisée en ce qu'**elle comprend 9,5 g de gluco-oligosaccharide.

8. Composition nutraceutique et/ou pharmaceutique pour son utilisation selon l'une quelconque des revendications précédentes, **caractérisée en ce qu'**elle se présente sous la forme de comprimés ou de gélules administrés une fois par jour.

9. Composition nutracetique et/ ou pharmaceutique pour son utilisation selon l'une quelconque des revendications précédentes pour réduire ou traiter une colopathie fonctionnelle.

10. Composition nutracetique et/ ou pharmaceutique pour son utilisation selon l'une quelconque des revendications précédentes à titre de complément alimentaire.

## Patentansprüche

1. Nutrazeutische und/oder pharmazeutische Zusammensetzung, welche dafür vorgesehen ist, die Produktion von Beta-Defensinen vom Typ 2 zu stimulieren, **dadurch gekennzeichnet, dass** sie als einzigen Wirkstoff ein Gluco-Oligosaccharid aufweist, welches der folgenden oligomeren Grundstruktur entspricht: 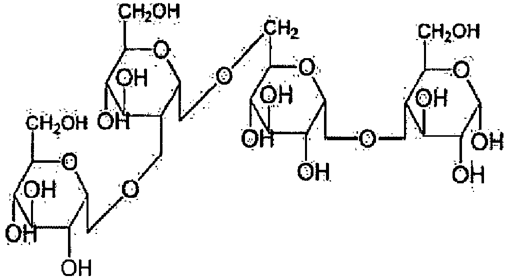 für ihre Anwendung bei der Behandlung von Darmentzündungen.

2. Nutrazeutische und/oder pharmazeutische Zusammensetzung für ihre Anwendung nach Anspruch 1, **dadurch gekennzeichnet, dass** sie zwischen 0,5 und 10 g Gluco-Oligosaccharid aufweist.

3. Nutrazeutische und/oder pharmazeutische Zusammensetzung für ihre Anwendung nach Anspruch 2, **dadurch gekennzeichnet, dass** sie 1 g Gluco-Oligosaccharid aufweist.

4. Nutrazeutische und/oder pharmazeutische Zusammensetzung für ihre Anwendung nach Anspruch 2, **dadurch gekennzeichnet, dass** sie 2,5 g Gluco-Oligosaccharid aufweist.

5. Nutrazeutische und/oder pharmazeutische Zusammensetzung für ihre Anwendung nach Anspruch 2, **dadurch gekennzeichnet, dass** sie 4 g Gluco-Oligosaccharid aufweist.

6. Nutrazeutische und/oder pharmazeutische Zusammensetzung für ihre Anwendung nach Anspruch 2, **dadurch gekennzeichnet, dass** sie 6 g Gluco-Oligosaccharid aufweist.

7. Nutrazeutische und/oder pharmazeutische Zusammensetzung für ihre Anwendung nach Anspruch 2, **dadurch gekennzeichnet, dass** sie 9,5 g Gluco-Oligosaccharid aufweist.

8. Nutrazeutische und/oder pharmazeutische Zusammensetzung für ihre Anwendung nach irgendeinem der vorhergehenden Ansprüche, **dadurch gekennzeichnet, dass** sie sich in Form von ein Mal pro Tag verabreichten Tabletten oder Kapseln darstellt.

9. Nutrazeutische und/oder pharmazeutische Zusammensetzung für ihre Anwendung nach irgendeinem der vorhergehenden Ansprüche, um eine funktionellen Kolopathie zu verringern oder zu behandeln.

10. Nutrazeutische und/oder pharmazeutische Zusammensetzung für ihre Anwendung nach irgendeinem der vorhergehenden Ansprüche als Nahrungsergänzungsmittel.

## Claims

1. A nutraceutical and/or pharmaceutical composition designed to stimulate the production of beta-defensins-2, **characterised in that** it comprises as a sole active ingredient a gluco-oligosaccharide satisfying the following basic oligomeric structure: 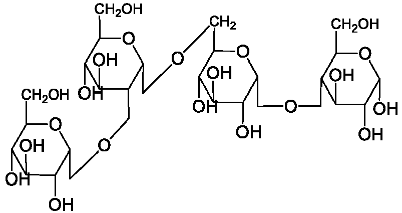 for it use in treatment of intestinal inflammations.

2. The nutraceutical and/or pharmaceutical composition for it use according to claim 1, **characterised in that** it comprises in the range 0.5 g to 10 g of gluco-oligosaccharide.

3. The nutraceutical and/or pharmaceutical composition for it use according to claim 2, **characterised in that** it comprises 1 g of gluco-oligosaccharide.

4. The nutraceutical and/or pharmaceutical composition for it use according to claim 2, **characterised in that** it comprises 2.5 g of gluco-oligosaccharide.

5. The nutraceutical and/or pharmaceutical composition for it use according to claim 2, **characterised in that** it comprises 4 g of gluco-oligosaccharide.

6. The nutraceutical and/or pharmaceutical composition for it use according to claim 2, **characterised in that** it comprises 6 g of gluco-oligosaccharide.

7. The nutraceutical and/or pharmaceutical composition for it use according to claim 2, **characterised in that** it comprises 9.5 g of gluco-oligosaccharide.

8. The nutraceutical and/or pharmaceutical composition for it use according to any one of the preceding claims, **characterised in that** it is in the form of tablets or of capsules administered as a dose once per day.

9. Nutraceutical and/or pharmaceutical composition for it use according to any one of the preceding claims for reducing or treating a functional colopathy.

10. Nutraceutical and/or pharmaceutical composition for it use according to any one of the preceding claims as a food supplement.
